# EUROPÄISCHE PATENTANMELDUNG

(11) **EP 2 476 381 A1**
(43) Veröffentlichungstag der Anmeldung: **18.07.2012**
(21) Anmeldenummer: 11000220.1
(22) Anmeldetag: 13.01.2011
(51) Int. Cl.: A61B 17/29, A61B 17/295

(54) **Zerlegbares chirurgisches Instrument mit flachem Kopf**

(71) Anmelder: Eberle GmbH & Co. KG, 75449 Wurmberg (DE)
(72) Erfinder: Löffler, Heiko, 75449 Wurmberg (DE)
(74) Vertreter: Schaeberle, Steffen

(57) **Zusammenfassung**

Die Erfindung betrifft ein zerlegbares chirurgisches Instrument, umfassend ein Außenrohr (2) mit einer an einem Endbereich (20) angeordneten Außenklinge (7), ein hohles Innenrohr (3), welches im Außenrohr (2) linear verschiebbar angeordnet ist, wobei an einem Ende des Innenrohrs ein Schubkopf (30) angeordnet ist, eine bewegliche Schneidbacke 5, welche am Endbereich (20) des Außenrohrs (2) angeordnet ist und lösbar mit dem Schubkopf in Eingriff bringbar ist und eine zwischen dem Endbereich (20) und der beweglichen Schneidbacke (5) angeordnete Führungskulisse (11) mit wenigstens einem vorstehenden Bereich (12) und wenigstens einer Ausnehmung (13), welche sich mit dem vorstehenden Bereich (12) im Eingriff befindet, wobei die Führungskulisse (11) eine über den Schubkopf (30) auf die bewegliche Schneidbacke (5) ausgeübte Linearkraft in eine Drehbewegung der Schneidbacke (5) umwandelt, und wobei zwischen dem Innenrohr (3) und der Schneidbacke (5) eine lösbare Verbindung (8) vorgesehen ist.

## Beschreibung

Die vorliegende Erfindung betrifft ein zerlegbares chirurgisches Instrument mit flachem Kopf, insbesondere zur minimalinvasiven Gelenkchirurgie.

Chirurgische Instrumente sind aus dem Stand der Technik in unterschiedlichen Ausgestaltungen bekannt. Derartige chirurgische Instrumente werden beispielsweise bei endoskopischen Eingriffen, z.B. an Gelenken, verwendet. Ein Problemkreis bei derartigen chirurgischen Instrumenten ist die nach Gebrauch notwendige vollständige Reinigung. Aus der EP 2 241 273 A1 ist beispielsweise ein zerlegbares, handbetätigtes chirurgisches Instrument bekannt, bei dem ein Innenrohr von einem Außenrohr getrennt werden kann, um eine separate Reinigung der Teile vorzunehmen. Eine Verbindung zwischen dem Innenrohr und einer am Außenrohr drehbar angeordneten Schneidbacke erfolgt dabei über eine Verzahnung mit einer Zahnstange und einem Zahnradabschnitt. Dieses chirurgische Instrument hat sich grundsätzlich bewährt, jedoch ist aufgrund der Verzahnung eine Aufbauhöhe des Kopfes des chirurgischen Instruments relativ dick. Um ein möglichst exaktes Arbeiten mit dem chirurgischen Instrument auch bei kleinen Gelenken zu ermöglichen, wäre es daher wünschenswert, dass ein Kopf des chirurgischen Instruments möglichst flach ausgebildet ist.

Es ist daher Aufgabe der vorliegenden Erfindung, ein zerlegbares chirurgisches Instrument bereitzustellen, welches bei einfachem Aufbau und einfacher kostengünstiger Herstellbarkeit einen möglichst flachen Kopfbereich aufweist und eine sichere Reinigung ermöglicht.

Diese Aufgabe wird durch ein chirurgisches Instrument mit den Merkmalen des Anspruchs 1 gelöst. Die Unteransprüche zeigen bevorzugte Weiterbildungen der Erfindung.

Das zerlegbare chirurgische Instrument mit den Merkmalen des Anspruchs 1 weist den Vorteil auf, dass es einen sehr flach bauenden Kopfbereich aufweist. Hierdurch können chirurgische Eingriffe auch an beengten Körperstellen vorgenommen werden. Dies wird erfindungsgemäß dadurch erreicht, dass das chirurgische Instrument ein Außenrohr und ein im Außenrohr linear verschiebbares hohles Innenrohr mit einem Schubkopf aufweist und am Außenrohr eine Außenklinge und eine bewegliche Schneidbacke angeordnet ist. Die bewegliche Schneidbacke ist dabei mit dem Schubkopf über eine lösbare Verbindung verbunden. Dabei ist zwischen der Außenklinge und der beweglichen Schneidbacke eine Führungskulisse mit einem vorstehenden Bereich und einer Ausnehmung angeordnet, wobei die Führungskulisse eine über den Schubkopf auf die bewegliche Schneidachse ausgeübte Linearkraft in eine Drehbewegung der beweglichen Schneidbacke umwandelt. Hierdurch ist es möglich, dass die Schneidbacke mittels der Führungskulisse die gewünschte Drehbewegung ausführt, um einen Schneidvorgang zum Abtrennen von Gewebe oder dgl. auszuführen. Durch die Verwendung der Führungskulisse kann auf eine an der Schneidbacke angeordnete Drehachse verzichtet werden, so dass ein flacherer Aufbau eines Kopfbereichs des chirurgischen Instruments möglich ist. Trotzdem bleibt die gewünschte Zerlegbarkeit des chirurgischen Instruments erhalten, so dass das chirurgische Instrument nach Gebrauch schnell und einfach gereinigt werden kann.

Ein besonders einfacher und kostengünstiger Aufbau ergibt sich, wenn der vorstehende Bereich der Führungskulisse durch zwei an einem Endbereich angeordnete Führungsbögen und die Ausnehmung der Führungskulisse komplementär zu den Führungsbögen ausgebildete Führungsnuten in der beweglichen Schneidbacke sind.

Die lösbare Verbindung zwischen der Schneidbacke und dem Innenrohr umfasst vorzugsweise einen T-förmigen Eingriffsbereich und eine T-förmige Ausnehmung. Besonders bevorzugt ist dabei der T-förmige Eingriffsbereich am Schubkopf des Innenrohrs angeordnet und die T-förmige Ausnehmung ist an der beweglichen Schneidbacke angeordnet. Besonders bevorzugt ist dabei der T-förmige Eingriffsbereich am Schubkopf einstückig mit dem Innenrohr gebildet.

Für einen besonders flachen Aufbau ist vorzugsweise ein Mittelpunkt, um welchen sich die Schneidbacke dreht, außerhalb des chirurgischen Instruments angeordnet.

Weiter bevorzugt umfasst die Schneidbacke einen Sicherungsstift, welcher die Schneidbacke bei einer gelösten Verbindung der Schneidbacke zum Innenrohr an der Außenklinge hält.

Der Sicherungsstift ist weiter bevorzugt derart ausgebildet, dass er eine Bewegung der Schneidbacke an einem an der Außenklinge vorgesehenen Anschlag begrenzt.

Weiter bevorzugt ist der Sicherungsstift in einer Bohrung in der Schneidbacke angeordnet, welche einen zu einer Oberseite der Schneidbacke offenen Schlitz aufweist. Dadurch nimmt der Sicherungsstift nur einen minimalen Raum an der Schneidbacke ein, so dass die Schneidbacke sehr flach gestaltet werden kann.

Weiter bevorzugt umfasst das chirurgische Instrument einen Handgriff, an welchem das Innenrohr und das Außenrohr lösbar befestigt sind. Am Handgriff ist vorzugsweise ein Anschluss zur Verbindung mit einem Unterdruckbereich angeordnet, so dass Material, welches durch das chirurgische Instrument abgetrennt wurde, durch das hohle Innenrohr abgesaugt werden kann.

Nachfolgend wird ein bevorzugtes Ausführungsbeispiel der Erfindung unter Bezugnahme auf die begleitende Zeichnung im Detail beschrieben. In der Zeichnung ist:
- Fig. 1: eine schematische, perspektivische Ansicht eines chirurgischen Instruments in vollständig geöffnetem Zustand,
- Fig. 2: eine schematische Schnittansicht des chirurgischen Instruments von Fig. 1,
- Fig. 3: eine perspektivische Ansicht des chirurgischen Instruments in teilweise geöffnetem Zustand,
- Fig. 4: eine schematische Schnittansicht des chirurgischen Instruments von Fig. 3,
- Fig. 5: eine perspektivische Ansicht des chirurgischen Instruments in geschlossenem Zustand,
- Fig. 6: eine schematische Schnittansicht des chirurgischen Instruments von Fig. 5,
- Fig. 7 und 8: perspektivische Ansichten der Schneidbacke,
- Fig. 9: eine perspektivische Ansicht eines Schubkopfs am Innenrohr,
- Fig. 10: eine perspektivische Ansicht einer Außenklinge am Außenrohr, und
- Fig. 11: eine Draufsicht von Fig. 10.

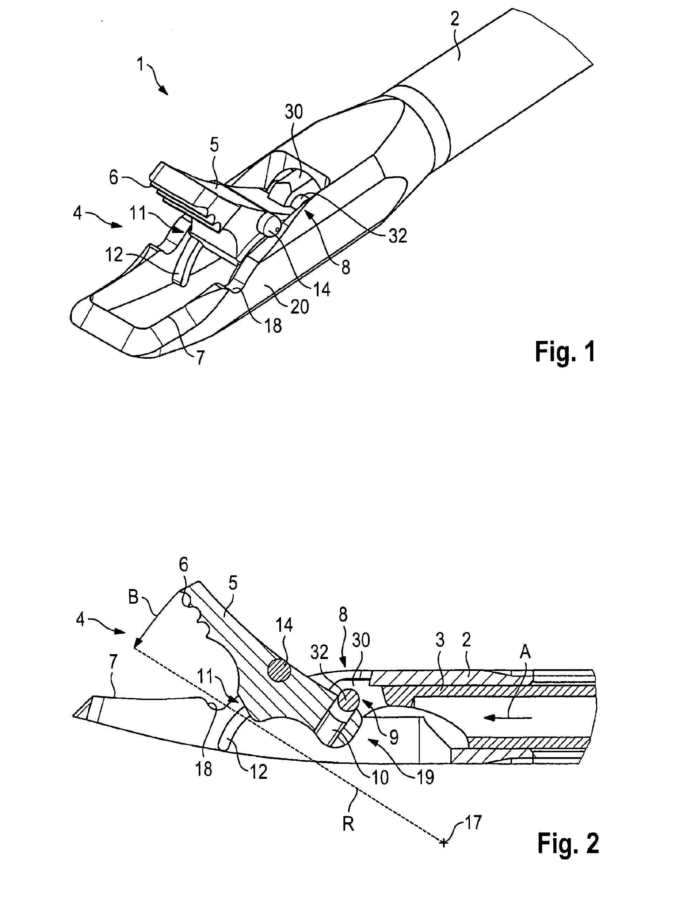

Nachfolgend wird unter Bezugnahme auf die Fig. 1 bis 11 ein zerlegbares chirurgisches Instrument 1 gemäß einem bevorzugten Ausführungsbeispiel der Erfindung im Detail beschrieben.

Wie aus Fig. 1 ersichtlich ist, umfasst das erfindungsgemäße chirurgische Instrument, welches in diesem Ausführungsbeispiel als handbetätigtes chirurgisches Instrument ausgebildet ist, ein hohles Außenrohr 2 und ein im Außenrohr angeordnetes hohles Innenrohr 3. Am Kopfbereich des chirurgischen Instruments 1 ist eine Schneidvorrichtung 4 mit einer beweglichen Schneidbacke 5 und einer umlaufenden Außenklinge 7 vorgesehen. Bei einem chirurgischen Eingriff abgetrenntes Material kann dabei durch das hohle Innenrohr 3 durch das chirurgische Instrument hindurch abgesaugt werden.

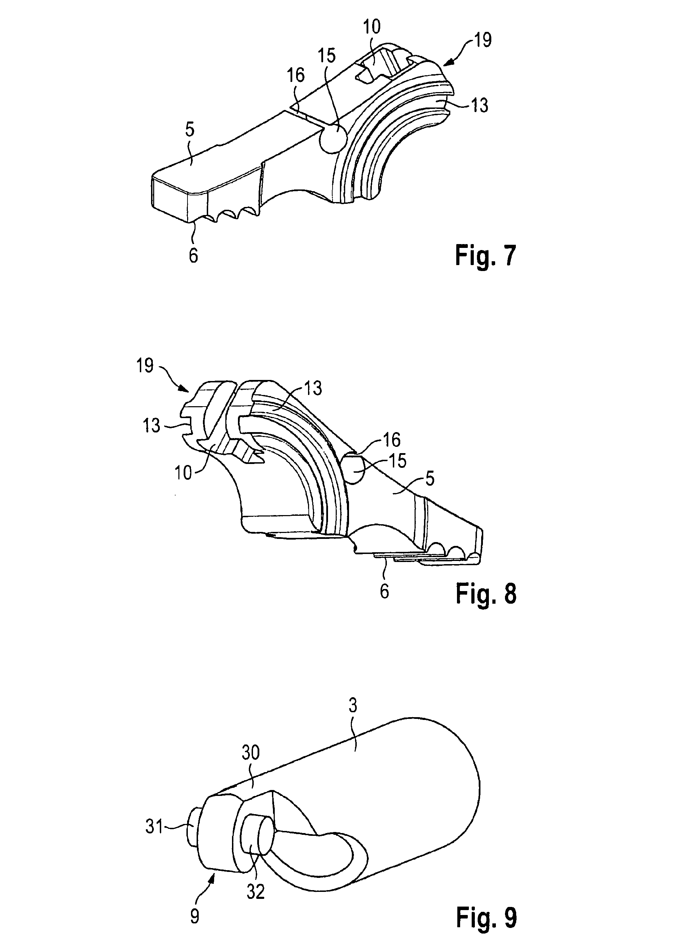

Die bewegliche Schneidbacke 5 ist mittels einer lösbaren Verbindung 8 am Innenrohr 3 befestigt. Die lösbare Verbindung 8 umfasst einen am Ende des Innenrohrs angeordneten T-förmigen Eingriffsbereich 9 mit einem Schubkopf 30 und zwei seitlich am Schubkopf angeordneten Zylinderabschnitten 31, 32 (vgl. Fig. 9). Das Innenrohr und somit der T-förmige Eingriffsbereich 9 können dabei in axialer Richtung im hohlen Außenrohr bewegt werden. Die bewegliche Schneidbacke 5 ist am Außenrohr angeordnet und umfasst mehrere Schneiden 6, welche in Wirkverbindung mit der umlaufenden Außenklinge 7 während eines chirurgischen Eingriffs Material abtrennen.

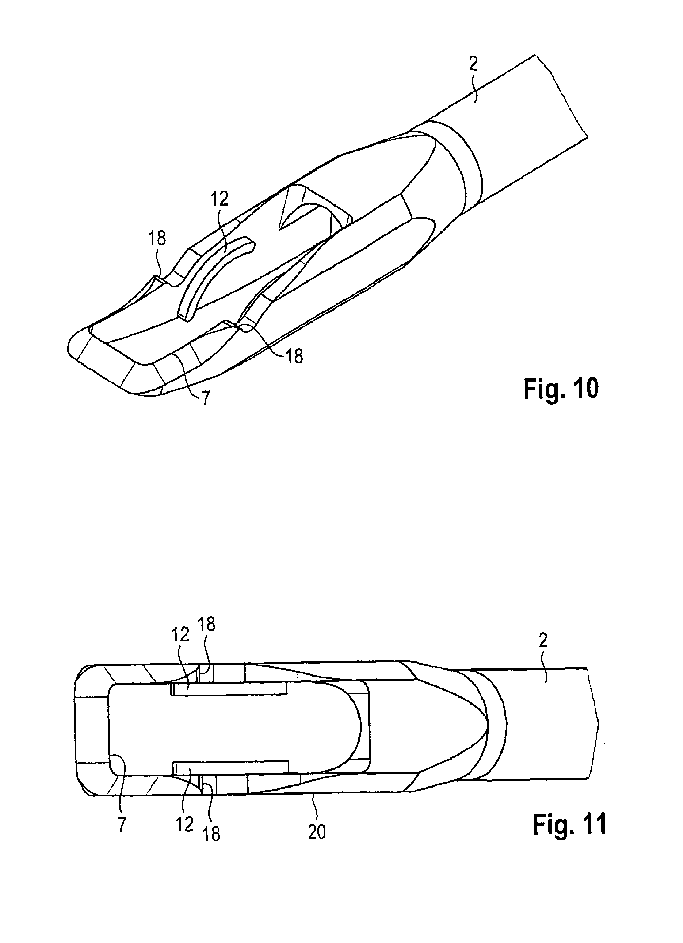

Zwischen der beweglichen Schneidbacke 5 und einem Endbereich 20 des Außenrohrs 2 ist eine Führungskulisse 11 vorgesehen. Hierbei sind am Endbereich 20 zwei vorstehende, bogenförmige Bereiche 12 vorgesehen (vgl. Fig. 10 und 11) und an der beweglichen Schneidbacke 5 zwei entsprechend den vorstehenden Bereichen 12 gebildete bogenförmige Ausnehmungen 13 vorgesehen (vgl. Fig. 7 und 8). Ferner weist die bewegliche Schneidbacke 5 eine T-förmige Ausnehmung 10 an einem zum Innenrohr gerichteten Endbereich 19 auf, welcher mit dem T-förmigen Eingriffsbereich 9 am Ende des Innenrohrs 3 in Eingriff bringbar ist. In der in Fig. 1 gezeigten vollständig geöffneten Position der beweglichen Schneidbacke 5 kann dabei der T-förmige Eingriffsbereich 9 von der T-förmigen Ausnehmung 10 außer Eingriff gebracht werden.

Durch die Führungskulisse 11 kann somit eine axiale Bewegung des hohlen Innenrohrs über den T-förmigen Eingriffsbereich 9 und die T-förmige Ausnehmung 10 in eine Drehbewegung der beweglichen Schneidbacke 5 überführt werden. Es sei angemerkt, dass die Führungskulisse 11 auch derart vorgesehen sein kann, dass der vorstehende Bereich an der Schneidbacke 5 und die Ausnehmung am Endbereich 20 des Außenrohrs vorgesehen sein kann.

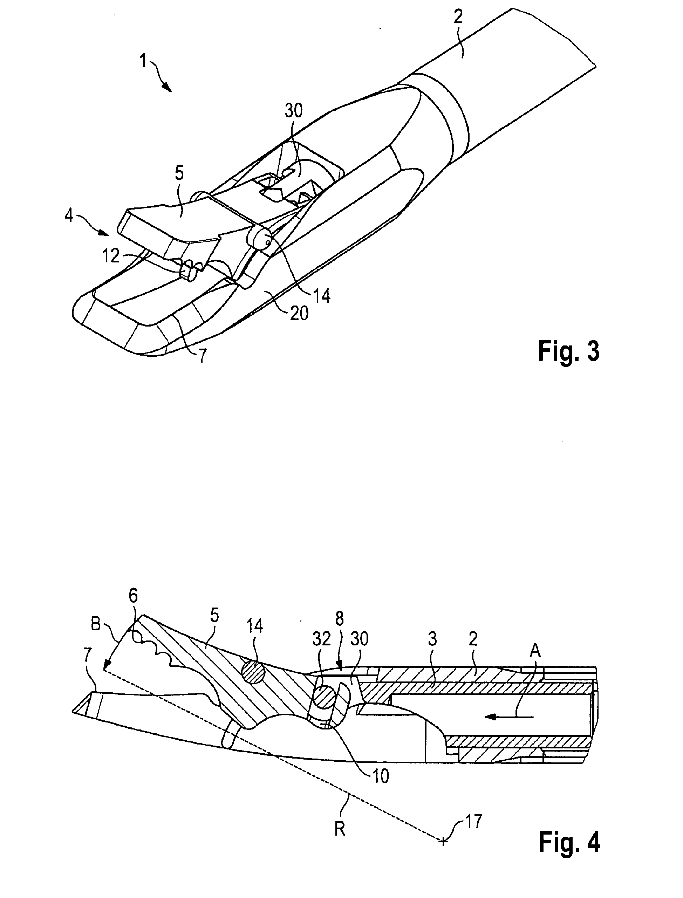

Ein Drehpunkt der beweglichen Schneidbacke 5 liegt dabei außerhalb des chirurgischen Instruments 1 und ist mit dem Bezugszeichen 17 (vgl. Fig. 2 und 4) bezeichnet. Dadurch schwenkt die bewegliche Schneidbacke 5 mit einem Radius R um den Drehpunkt 17.

Um ein Verlieren der beweglichen Schneidbacke 5 vom Endbereich 20 des Außenrohrs 2 zu verhindern, ist an der beweglichen Schneidbacke 5 zusätzlich noch ein Sicherungsstift 14 vorgesehen. Der Sicherungsstift 14 ist dabei in einer in der Schneidbacke 5 angebrachten Querbohrung 15 angeordnet. Die Querbohrung 15 ist dabei derart vorgesehen, dass zur Oberseite der Schneidbacke ein durchgehender Schlitz 16 gebildet ist. Am Endbereich 20 des Außenrohrs ist ferner noch ein Anschlag 18 vorgesehen, welcher mit dem Sicherungsstift 14, welcher zu beiden Seiten der beweglichen Schneidbacke 5 vorsteht, eine Drehbewegung der Schneidbacke 5 begrenzt. Somit weist der Sicherungsstift 14 neben der Sicherungsfunktion noch eine Funktion einer Definition einer Endstellung der Schneidvorrichtung 4 auf.

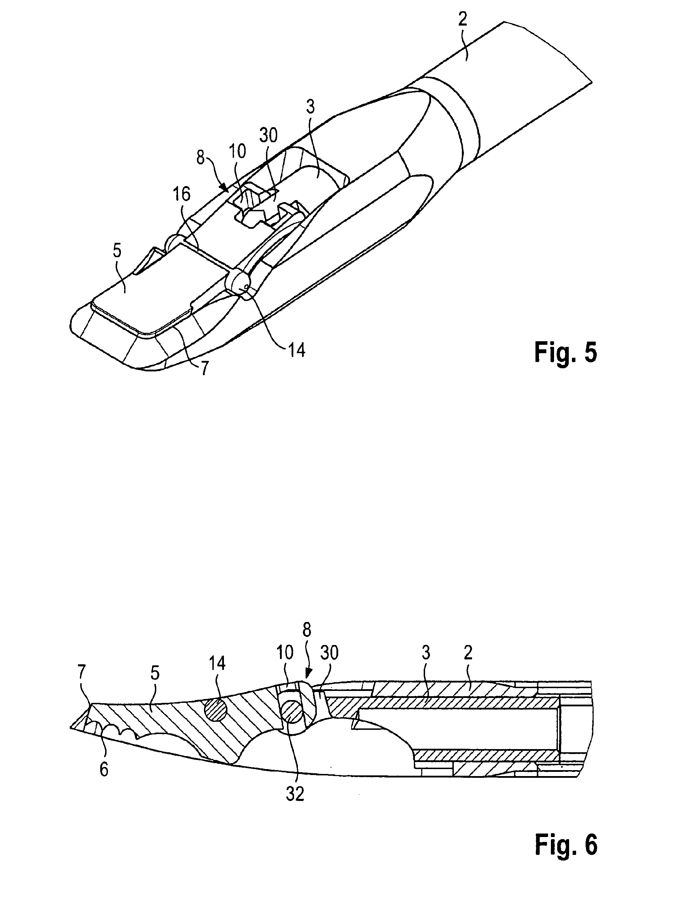

Die Funktion des erfindungsgemäßen chirurgischen Instruments ist dabei wie folgt. Durch Betätigung an einem nicht gezeigten Handgriff wird das hohle Innenrohr 3 linear in Richtung des Pfeils A (vgl. Fig. 2 und 4) bewegt. Diese lineare Bewegung wird über den T-förmigen Eingriffsbereich 9 und die T-förmige Ausnehmung 10 sowie die Führungskulisse 11 in eine Drehbewegung der Schneidbacke 5 überführt. In der Zeichnung zeigen die Fig. 1 und 2 den vollständig geöffneten Zustand der Schneidvorrichtung, die Fig. 3 und 4 einen Zustand während des Schließvorgangs und die Fig. 5 und 6 den geschlossenen Zustand der Schneidvorrichtung. Die bewegliche Schneidbacke führt dabei eine Drehbewegung in Richtung des Pfeils B (vgl. Fig. 2 und 4) aus.

Fig. 2 zeigt dabei die vollständige geöffnete Position des chirurgischen Instruments in einer Schnittdarstellung, bei der die Zylinderabschnitte 31, 32 am Schubkopf 30 genau auf einer Kante der T-förmigen Ausnehmung 10 liegen. In dieser Position kann die Verbindung zwischen dem Innenrohr und der Schneidbacke 5 gelöst werden. Hierzu muss das Innenrohr 3 lediglich in entgegengesetzter Richtung zur Schubrichtung A aus dem Außenrohr herausgezogen werden.

Durch die erfindungsgemäße Lösung mit der Führungskulisse 11 zwischen der Schneidbacke 5 und dem Endbereich 20 des Außenrohrs 2 kann dabei ein besonders flacher Aufbau des Kopfes des chirurgischen Instruments realisiert werden. Trotzdem ist eine Lösbarkeit des Innenrohrs 3 vom Außenrohr 2 möglich, was eine Reinigung des chirurgischen Instruments sehr erleichtert. Die Flachheit des Aufbaus wird insbesondere aus Fig. 6 ersichtlich, wobei der gesamte Kopfbereich des chirurgischen Instruments nicht größer als ein Außendurchmesser des Außenrohrs 2 ist. Trotzdem kann erfindungsgemäß eine exzellente Kraftübertragung vom Innenrohr 3 auf die bewegbare Schneidbacke 5 realisiert werden. Die lösbare Verbindung 8 zwischen Innenrohr 3 und Schneidbacke 5 stellt eine formschlüssige Verbindung zwischen diesen Bauteilen bereit. Grundsätzlich sind auch andere geometrische Formen von formschlüssigen Verbindungen zwischen dem Innenrohr und der Schneidbacke möglich, jedoch ist die T-Form bevorzugt, da dadurch während des Schließens der Schneidbacke 5 eine gleichmäßige, zweiseitige Führung durch die beiden seitlichen Zylinderabschnitte 31, 32 möglich ist. Weiterhin kann das während des chirurgischen Eingriffs abgetrennte Material sicher durch das hohle Innenrohr 3 abgesaugt werden.

Das erfindungsgemäße chirurgische Instrument eignet sich insbesondere als handbetätigtes Instrument für chirurgische Eingriffe an Gelenken.

### Bezugszeichenliste

- 1: Chirurgisches Instrument
- 2: hohles Außenrohr
- 3: hohles Innenrohr
- 4: Schneidvorrichtung
- 5: bewegliche Schneidbacke
- 6: Schneide
- 7: umlaufende Außenklinge
- 8: lösbare Verbindung
- 9: T-förmiger Eingriffsbereich
- 10: T-förmige Ausnehmung
- 11: Führungskulisse
- 12: vorstehender Bereich
- 13: Ausnehmung
- 14: Sicherungsstift
- 15: Bohrung
- 16: Schlitz
- 17: Drehpunkt
- 18: Anschlag
- 19: Endbereich
- 20: Endbereich
- 30: Schubkopf
- 31: Zylinderabschnitt
- 32: Zylinderabschnitt
- A: Linearrichtung
- B: Drehrichtung
- R: Radius

## Patentansprüche

1. Zerlegbares chirurgisches Instrument, umfassend
- ein Außenrohr (2) mit einer an einem Endbereich (20) angeordneten Außenklinge (7),
- ein hohles Innenrohr (3), welches im Außenrohr (2) linear verschiebbar angeordnet ist, wobei an einem Ende des Innenrohrs ein Schubkopf (30) angeordnet ist,
- eine bewegliche Schneidbacke (5), welche am Endbereich (20) des Außenrohrs (2) angeordnet ist und lösbar mit dem Schubkopf (30) in Eingriff bringbar ist und
- eine zwischen dem Endbereich (20) und der beweglichen Schneidbacke (5) angeordnete Führungskulisse (11) mit wenigstens einem vorstehenden Bereich (12) und wenigstens einer Ausnehmung (13), welche sich mit dem vorstehenden Bereich (12) im Eingriff befindet,
- wobei die Führungskulisse (11) eine über den Schubkopf (30) auf die bewegliche Schneidbacke (5) ausgeübte Linearkraft in eine Drehbewegung der Schneidbacke (5) umwandelt, und
- wobei zwischen dem Innenrohr (3) und der Schneidbacke (5) eine lösbare Verbindung (8) vorgesehen ist.

2. Instrument nach Anspruch 1, **dadurch gekennzeichnet, dass** der vorstehende Bereich (12) der Führungskulisse (11) zwei am Endbereich (20) gebildete vorstehende Führungsbögen umfasst und die Ausnehmung (13) zwei komplementär zu den Führungsbögen ausgebildete Führungsnuten in der Schneidbacke (5) umfasst.

3. Instrument nach einem der vorhergehenden Ansprüche , **dadurch gekennzeichnet, dass** die lösbare Verbindung (8) zwischen der Schneidbacke (5) und dem Innenrohr (3) einen T-förmigen Eingriffsbereich (9) und eine T-förmige Ausnehmung (10) umfasst.

4. Instrument nach Anspruch 3, **dadurch gekennzeichnet, dass** der T-förmige Eingriffsbereich (9) am Ende des Innenrohrs (3) angeordnet ist und die T-förmige Ausnehmung (10) am zum Innenrohr gerichteten Ende der Schneidbacke (5) angeordnet ist.

5. Instrument nach Anspruch 4, **dadurch gekennzeichnet, dass** der T-förmige Eingriffsbereich (9) einstückig mit dem Innenrohr gebildet ist.

6. Instrument nach einem der vorhergehenden Ansprüche, **dadurch gekennzeichnet, dass** ein Drehpunkt (17) der Schneidbacke (5) außerhalb des chirurgischen Instruments liegt.

7. Instrument nach einem der vorhergehenden Ansprüche, **dadurch gekennzeichnet, dass** die Schneidbacke (5) ferner einen Sicherungsstift (14) umfasst, welcher die Schneidbacke (5) am Endbereich (20) des Außenrohrs (2) hält.

8. Instrument nach Anspruch 7, **dadurch gekennzeichnet, dass** der Sicherungsstift (14) eine Bewegung der Schneidbacke (5) an einem am Endbereich (20) gebildeten Anschlag (18) begrenzt.

9. Instrument nach Anspruch 7 oder 8, **dadurch gekennzeichnet, dass** der Sicherungsstift (14) in einer Bohrung (15) in der Schneidbacke (5) angeordnet ist, welche an einer Oberseite der Schneidbacke einen offenen Schlitz (16) umfasst.

10. Instrument nach einem der Ansprüche 3 bis 5, **dadurch gekennzeichnet, dass** der T-förmige Eingriffsbereich (9) zwei seitliche Zylinderabschnitte (31, 32) umfasst.
